# EUROPEAN PATENT SPECIFICATION

(11) **EP 2 818 180 B1**
(45) Date of publication and mention of the grant of the patent: **11.07.2018**
(21) Application number: 12869453.6
(22) Date of filing: 15.05.2012
(51) Int. Cl.: A61K 38/21, A61P 27/02, A61K 9/00

(54) **ANTIVIRAL EYE DROPS**
ANTIVIRALE AUGENTROPFEN
GOUTTES OCULAIRES ANTIVIRALES

(30) Priority: 24.02.2012 RU 2012106474
(43) Date of publication of application: 31.12.2014
(73) Proprietor: Markov, Ilya Aleksandrovich, Moscow 121433 (RU); Markova, Elena Alekseevna, Moscow 121433 (RU); Gaponyuk, Polina Petrovna, Moscow 121433 (RU); Markova, Inna Nikolaevna, Moscow 119602 (RU)
(72) Inventor: MARKOV, Ilya Aleksandrovich, MOSCOW 121433 (RU); MARKOVA, Elena Alkseevna, MOSCOW 121433 (RU); GAPONYUK, Polina Petrovna, MOSCOW 121433 (RU); MARKOVA, Inna Nikolaevna, 119602, Moscow (RU); GAPONYUK, Petr Yakovlevich, deceased (RU)
(74) Representative: Jeck, Anton
(86) International application number: PCT/RU2012/000381
(87) International publication number: WO 2013/125977

(56) References cited:
- WO-A2-02/085248
- WO-A2-2008/074885
- RU-C1- 2 140 286
- US-A1- 2010 152 147
- US-A1- 2012 003 296
- 'Entsiklopediia lekarstv.' REGISTR LEKARSTVENNYKH SREDSTV ROSSII®. 2007, pages 681 - 682

## Description

### Technical Field

The invention refers to pharmaceutical industry, specifically to the development of interferon-containing compositions capable to increase their antiviral potency and applicable in ophthalmology for the treatment of viral diseases of the eye.

### Background

Infectious diseases play one of the key roles in ophthalmology. The spectrum of available anti-microbial agents is rather broad. It includes anti-viral agents - natural and synthetic compounds used for the prevention and therapy of viral diseases, e.g. modified nucleosides (idoxuridine, vidarabine, ribavirin, acyclovir, azidothymidine, etc), Acyclovir (Virolex, Zovirax, Medovir, Cyclovir), Ganciclovir (Cymevene), Zalcitabine (Hivid), Zidovudine (Retrovir), Idoxuridine (Kerecide, Stoxil, Emanil), Iso-propyluracil (Hevizos), Ribavirin (Virazole), Famciclovir (Famvir), Adamantane derivatives (Remandadine, Adaprolin, Amantadine), tiosemicarbazone derivatives, interferon inducers (Gossypol, Poludan, Cycloferon), and agents with virucidal effect (Bonafton, Oxoline, Tebrofen, Florinal).

However, there are still many challenges in the pharmacotherapy of viral ocular diseases. The reason is a lack of specific agents for the treatment of viral infections in general and ocular viral diseases in particular.

Considerable efficacy of natural interferon derivatives for topical application has been demonstrated in the treatment of such diseases as conjunctivitis - adenoviral, epidemic, hemorrhagic (caused by enteroviruses) and herpetic; keratitis - adenoviral, herpetic (vesicular, punctuate, dendritic, geographic); keratitis - herpetic stromal keratitis with and without ulceration; keratoconjunctivitis caused by herpes virus and adenovirus; herpetic uveitis; and, herpetic keratouveitis (with and without ulceration).

In our country, such diseases are treated with Locferon which contains interferon from donor leucocytes. Its production is induced by a virus (parainfluenza virus type 1 (Sendai virus); State Collection of Viruses # "2339). Micro- and ultrafiltration methods are used to concentrate and purify the product (RU, Patent 2108804, A 61 K 38/21, 1998).

However, natural-origin interferons for which the human blood is used as a source material are not completely safe as regards their contamination with viruses (hepatitis viruses, HIV, CMV and herpes viruses).

In view of the above, health professionals use gene-engineered interferons in their practice more commonly, since these products are characterized by the highest extent of purification (up to 98%) and safety with respect to viral contamination.

A pharmaceutical anti-viral composition for making eye drops is also known; it contains human interferon, biocompatible polymer - 6% polyglucin solution and buffer solution with the following concentration of ingredients per ml of the solution:
Interferon, IU - (1-6.6)*10
Biocompatible polymer (polyglucin) - 5 - 30
Buffer solution - to bring pH of the solution to 7.0 - 7.6
(RU, Patent 2095081, Class A 61 K 38/21, 1997)

There is another known drug with antiviral and antimicrobial activity "INTERLYSIN" containing the following concentrations of human liquid leucocytic interferon and lysozyme per ml:

| | |
|---|---|
| Interferon - | 2500-5000 IU/ml |
| Lysozyme - | 2.5-5.0 mg/ml. |

(RU, Patent 2188665, Class A61K38/19, 2001)

Its disadvantage is associated with the use of human interferon, as no guarantees can be provided that the product is free from viral contaminants.

Berofor manufactured by BOEHRINGER INGELHEIM (Austria) is designed for the application as eye drops. The product is made using recombinant DNA-technology with *E.coli* strains (F. I. Ershov. Interferon System in the Normal and Abnormal Condition. M.:Meditsina. 1996, p. 216).

Another available anti-viral agent contains fucoidan derived from the brown algae, namely fucus or laminaria genera. It is presented in the form of saline solution with the molecular weight of max. 30 kDa and contains a variety of plant polysaccharides in the concentration of 0.01-15%, including fucoidan, laminaran and alginate derived from the brown algae (focus or laminaria genus). Saline solution contains a preservative from the following group: sodium benzoate in the concentration of 0.01-0.3% or potassium sorbate 0.02-0.3%, or formaldehyde in the concentration of 0.01-0.3%. As a saline, it contains sodium chloride in the concentration of 0.85-0.95% in the water solution (the rest). The variety of plant-origin polysaccharides includes fucoidan, laminaran and alginate in randomly selected ratios. In the preferred composition of plant polysaccharides, the concentration of fucoidan makes up 10-99.9% (RU, Patent 2381807, Class A61K36/03, 2008).

This product has such disadvantages as low efficacy and potential allergic reactions.

Reaferon, recombinant alpha-interferon, is another available agent produced by *Pseudomonas* strain with human leucocytic alpha-interferon gene inserted in its genome. It has anti-viral, immunomodulating and anti-cancer effects. Reaferon is dissolved in the normal saline solution and used for the treatment of conjunctivitis, keratoconjunctivitis and keratitis in the form of eye drops (2 drops are instilled 6-8 times a day and then 3-4 times a day for 2 weeks). One ampoule is diluted in 10 ml of saline. The diluted product can be used only during 24 hours (M. D. Mashkovsky. Pharmaceutical Products. M.: Meditsina. 1993, Volume 2, p.p. 390-391).

The closest analog of this invention with respect to its proposed nature and achievable results, is an anti-viral agent - eye drops "Ophthalmoferon," containing recombinant interferon; stabilizer of biological and physicochemical properties selected from the group - polyvinyl pyrrolidone, polyvinyl alcohol, methylcellulose derivative (Na carboxymethyl cellulose); stabilizer of the resistance to microbial contamination selected from the group - Nipagin, quaternary ammonium compounds, chlorhexidine; anti-oxidizing agent; diphenylhydramine (Dimedrol); and, buffer mixture composing of borate acetate or phosphate buffer (RU, 2140286, A 61 K 38/21, 1999).

The prototype has such a drawback as short drug-eye cornea contact time. As a result, the eye drops should be instilled for multiple times and the duration of treatment should be longer.

Clinical experience gained for intereferons over the last several years has demonstrated that their efficacy can be enhanced through the application of certain formulations (based on the pathogenetic features of specific diseases) to reveal their anti-viral and immunomodulating action and at the same time to avoid cytostatic or other side effects. Thus, it is practical to develop various formulations of the eye drops.

### Disclosure of Invention

The object of the invention is to develop eye drops with enhanced anti-viral, immunomodulatory, antihistamine and regenerating activity, improved penetration capability and sustained-release action that will facilitate the recovery of transparent corneal stroma in patients with viral ocular diseases.

Technical result of the invention is an increase of therapeutic efficiency of the claimed eye drops due to their sustained release action by prolonging drug-corneal contact time that ensures shorter therapeutic course and less complication rates vs. prototype.

To achieve the above technical result, the eye drops composing of recombinant interferon, stabilizer of biological and physicochemical properties, stabilizer of the resistance to microbial contamination, anti-oxidizing agent and buffer solution, according to the invention, contain antihistamine products selected from the group: antazoline, azelastin, tetryzoline, and as a stabilizer of biological and physicochemical properties they contain agents selected from the group:
macrogol 400-12000, as a stabilizer of resistance to microbial contamination the eye drops contain agents selected from the group: sorbic acid, boric acid; in addition, they contain sodium chloride and/or sodium acetate with the following concentrations of components per ml of the solution:

| | |
|---|---|
| recombinant interferon, IU - | 500 - 10000000 |
| antihistamine agents, g | 0.0001 - 0.1 |
| stabilizer of biological, physicochemical properties, g | 0.0001 - 0.1 |
| stabilizer of resistance to microbial contamination, g | 0.0001 - 0.1 |
| anti-oxidizing agent, g | 0.0001 - 0.1 |
| sodium chloride, g | 0.0001 - 0.1 |
| and/or | |
| sodium acetate, g | 0.0001 - 0.1 |
| buffer mixture, ml | the rest |

The eye drops contain anti-oxidizing agent - Trilon B or buthylhydroxytoluene in the amount of 0.0001 - 0.1 g per ml of the solution.

The eye drops contain recombinant interferon selected from the group: alpha-, beta, gamma-interferon.

The eye drops contain buffer mixture - acetate borate or phosphate, or borate buffer.

### Embodiments of Invention

The essence of the invention is highlighted in the following examples.
(Reference) Example 1. The technology of production of eye drops "Ophthalmoferon." Take stabilizers: low-molecular polyvinylpyrolidone and Nipasol. Mix with Anthazoline. Add Trilon B, normal saline in phosphate buffer with pH 6.8-7.6. Mix and sterilize on the filters. Then, add alpha-recombinant interferon and mix. Finished products are packed in the appropriate containers, sealed and labeled. Take the above components in the following concentrations per ml of the solution:

| | Option 1 | Option 2 | Option 3 |
|---|---|---|---|
| Alpha-recombinant interferon, IU | 500 | 10000 | 10000000 |
| Polyvinylpyrolidone, g | 0.0001 | 0.001 | 0.1 |
| Nipasol, g | 0.0001 | 0.001 | 0.1 |
| Anthazoline, g | 0.0001 | 0.001 | 0.1 |
| Trilon B, g | 0.0001 | 0.001 | 0.1 |
| Sodium chloride, g | 0.0001 | 0.001 | 0.1 |
| Phosphate buffer, ml | the rest | | |

Example 2. Same as in Example 1, except the composition of components to be taken in the following concentrations per ml of the solution:

| | Option 1 | Option 2 | Option 3 |
|---|---|---|---|
| Beta-recombinant interferon, IU | 500 | 1000 | 10000000 |
| Macrogol 400, g | 0.0001 | 0.001 | 0.1 |
| Sorbic acid, g | 0.0001 | 0.001 | 0.1 |
| Azelastin, g | 0.0001 | 0.001 | 0.1 |
| Buthylhydroxytoluene, g | 0.0001 | 0.001 | 0.1 |
| Sodium acetate, g | 0.0001 | 0.001 | 0.1 |
| Borate buffer, ml | the rest | | |

Example 3: Same as in Example 1, except the composition of components to be taken in the following concentrations per ml of the solution:

| | Option 1 | Option 2 | Option 3 |
|---|---|---|---|
| Gamma-recombinant interferon, IU | 500 | 100000 | 10000000 |
| Macrogol 1200, g | 0.0001 | 0.001 | 0.1 |
| Boric acid, g | 0.0001 | 0.01 | 0.1 |
| Tetryzoline, g | 0.0001 | 0.001 | 0.1 |
| Trilon B, g | 0.0001 | 0.001 | 0.1 |
| Sodium chloride, g | 0.00005 | 0.005 | 0.05 |
| Sodium acetate, g | 0.00005 | 0.005 | 0.05 |
| Borate acetate buffer, ml | the rest. | | |

(Reference) Example 4. Same as in Example 1, except the composition of components to be taken in the following concentrations per ml of the solution:

| | Option 1 | Option 2 | Option 3 |
|---|---|---|---|
| Alpha-recombinant interferon, IU | 500 | 5000 | 10000000 |
| Beta-recombinant interferon, IU | 500 | 5000 | 10000000 |
| Propylene glycol, g | 0.0001 | 0.001 | 0.1 |
| Nipasol, g | 0.00005 | 0.0005 | 0.05 |
| Boric acid, g | 0.00005 | 0.0005 | 0.05 |
| Diphenhydramine hydrochloride, g | 0.0001 | 0.001 | 0.1 |
| Trilon B, g | 0.0001 | 0.001 | 0.1 |
| Sodium chloride, g | 0.0001 | 0.001 | 0.1 |
| Phosphate buffer, ml | - the rest. | | |

(Reference) Example 5. Same as in Example 1, except the composition of components to be taken in the following concentrations per ml of the solution:

| | Option 1 | Option 2 | Option 3 |
|---|---|---|---|
| Alpha-recombinant interferon, IU | 500 | 10000 | 10000000 |
| Gamma-recombinant interferon, IU | 500 | 10000 | 10000000 |
| Macrogol 400, g | 0.00005 | 0.001 | 0.05 |
| Macrogol 12000, g | 0.00005 | 0.001 | 0.05 |
| Sorbic acid, g | 0.0001 | 0.01 | 0.1 |
| Azelastin, g | 0.00005 | 0.001 | 0.05 |
| Diphenhydramine hydrochloride, g | 0.00005 | 0.001 | 0.05 |
| Butyl hydroxytoluene, g | 0.0001 | 0.01 | 0.1 |
| Sodium chloride, g | 0.0001 | 0.001 | 0.1 |
| Borate buffer, ml | the rest. | | |

The final product - eye drops "Ophthalmoferon" have an appearance of the transparent liquid.

Produced formulations were inspected to assess their purity and effectiveness. The assessed characteristics included pH value, transparency, optic rotation, moisturizing effect, surface tension, dynamic viscosity, a lack of corneal irritation and eye cornea-drug contact time.

Minor variations of pH values within 6.8 - 7.6 were found for the developed compositions of the eye drops.

Against the background of black color in diffuse light the transparency of the tested solutions of eye drops did not differ from the water transparency.

The measurement of surface tension of the eye drops by commonly used methods demonstrated that all the compositions possess surface activity and, as a result, have good fluidity on the cornea.

Refracting index of the developed eye drop compositions was relatively low, it did not cause visual blur and was close to the lachrymal fluid value (1.334).

The time of retention of the claimed eye drops on the cornea was determined in a group of rabbits. The study results showed that the extended release of the claimed eye drop compositions was two or three times higher vs. prototype due to their improved penetration in the ocular tissues. They also were found to have better coating effect, higher viscosity and selectivity for the eye surface vs. prototype.

Irritating and toxic effects of the eye drop compositions were assessed in experimental rabbits. The eye drops were instilled in the conjunctival sac, 1 or 2 drops, 5-8 times a day for two weeks. Hyperemia, edema or other conjunctival abnormalities were not found in the experimental study. In addition, no cases of animal death or acute intoxication were reported.

Using a model of mild non-infectious inflammation of the eye conjunctiva, a shorter duration of therapy of inflammatory ocular lesions was demonstrated in animals treated by instillation of the claimed eye drops.

### Industrial Applicability

Efficacy of the eye drops manufactured according to the provided examples was assessed in four groups of patients (each groups included 5 patients) with dry eye syndrome (DES) or dry keratoconjunctivitis.

The first (control) group of patients with diagnosed herpetic stromal keratitis of one eye received treatment consisting of the prototype agent and concomitant antibacterial therapy. Twenty days later their condition improved. The ocular symptoms relieved completely. The recovery of the corneal transparency on the both eyes was recorded one month after the beginning of the therapy.

The second group included patients with diagnosed herpetic stromal keratitis of the both eyes. Therapy administered to these patients included the claimed eye drops manufactured according to Example 1 (5 drops 6 times a day for two weeks). At the same time the patients received laevomycetin instillation. The prescribed therapy was found to improve such signs as lacrimation and photophobia. A week later the ocular irritation reduced and infiltrates in the corneal stroma decreased. By day 16 from the beginning of the therapy, the irritation disappeared and the corneal transparency fully recovered.

The third group of patients had the following diagnosis: adenoviral keratoconjunctivitis, severe hyperemia and edema of mucous membrane of one of the eyes. Punctate subepithalial infiltrates in the cornea. Recurrent corneal erosions. In addition to the routine antibiotic therapy, the patients received the claimed eye drops manufactured according to Example 2 based on the following scheme: 5-8 drops 3-4 times a day during two weeks. A positive effect was shown on day 4: the hyperemia reduced and the discomfort disappeared. After two weeks of treatment the corneal opacity resolved completely with full epithelialization. No recurrent corneal erosions were found during the follow-up visit 6 months later.

In Group 4 patients had diagnosis of adenoviral keratoconjunctivitis. They received instillations of Laevomycetin and the claimed eye drops prepared according to Example 3. On day 5, significant improvements were found: the reduction of photophobia and the dissolution of some of the subepithelial opacities. On day 14 of the treatment, the eye returned to the normal state and the cornea became completely transparent.

Review of the treatment outcome with the proposed eye drops has demonstrated positive therapeutic results in patient groups 2, 3 and 4, where neither side effects, nor allergic reactions developed during the application of the claimed eye drops.

The selection of eye drop components and their amounts provided an opportunity to achieve a new technical result - the demonstration of satisfactory anti-inflammatory effect and the prevention of pathological lesions in epithelial cells of the conjunctiva and cornea that can cause damage of the corneal stroma and development of erosions, ulcers and perforations.

Thus, the claimed eye drops were found to have improved therapeutic efficacy in the treatment of viral ocular infections due to their sustained release activity and, thus to reduce duration of the disease and to increase remission period up to 6 months in patients with chronic diseases.

## Claims

1. Ophthalmic composition comprising (in concentrations per ml of the solution):
| | |
|---|---|
| recombinant interferon (IU) | 500 - 10 000 000 |
| antihistamine agents (g) | 0.0001 -0.1 |
| stabilizer of biological and physicochemical properties (g) | 0.0001 - 0.1 |
| stabilizer of resistance to microbial contamination (g) | 0.0001 - 0.1 |
| anti-oxidizing agent (g) | 0.0001 - 0.1 |
| sodium chloride (g) and/or | 0.0001- 0.1 |
| sodium acetate (g) | 0.0001 - 0.1 |
| buffer solution (ml) | the rest; |
wherein
(i) the antihistamine agents are selected from anthazoline, azelastin, tetryzoline;
(ii) the stabilizers of biological and physicochemical properties, are selected from Macrogol 400-12000;
(iii) the stabilizer of resistance to microbial contamination are selected from sorbic acid, boric acid.

2. Ophthalmic composition comprising (in concentrations per ml of the solution):
| | |
|---|---|
| beta recombinant interferon (IU) | 500 - 10 000 000 |
| antihistamine agents (g) | 0.0001 - 0.1 |
| stabilizer of biological and physicochemical properties (g) | 0.0001 - 0.1 |
| stabilizer of resistance to microbial contamination (g) | 0.0001 - 0.1 |
| anti-oxidizing agent (g) | 0.0001 - 0.1 |
| sodium acetate (g) | 0.0001 - 0.1 |
| borate buffer solution (ml) | the rest; |
wherein
(i) the antihistamine agents is azelastin;
(ii) the stabilizer of biological and physicochemical properties, is Macrogol 400;
(iii) the stabilizer of resistance to microbial contamination is sorbic acid.

3. Ophthalmic composition comprising (in concentrations per ml of the solution):
| | |
|---|---|
| gamma recombinant interferon (IU) | 500 - 10 000 000 |
| antihistamine agents (g) | 0.0001 - 0.1 |
| stabilizer of biological and physicochemical properties (g) | 0.0001 - 0.1 |
| stabilizer of resistance to microbial contamination (g) | 0.0001 - 0.1 |
| anti-oxidizing agent (g) | 0.0001 - 0.1 |
| sodium chloride (g) and | 0.00005 - 0.05 |
| sodium acetate (g) | 0.00005 - 0.05 |
| borate buffer solution (ml) | the rest; |
wherein
(i) the antihistamine agent is tetryzoline;
(ii) the stabilizer of biological and physicochemical properties, is Macrogol 12000;
(iii)the stabilizer of resistance to microbial contamination is boric acid.

4. The composition according to any of claims 1 to 6, wherein the anti-oxidizing agent is Trilon B or butylhydroxytoluene, both in a concentration of 0.0001 - 0.1 g/ per ml of solution.

5. The composition according to any of claims 1 to 7, wherein the interferon is recombinant interferon selected from alpha-, beta-, gamma interferon.

6. The composition according to any one of claims 1 to 8, wherein the buffer solution is selected from phosphate buffer, borate buffer or borate acetate buffer.

## Patentansprüche

1. Ophthalmische Zusammensetzung, umfassend (in Konzentrationen pro ml der Lösung):
| | |
|---|---|
| rekombinantes Interferon (IE) | 500 - 10 000 000 |
| Antihistaminika (g) | 0,0001 - 0,1 |
| Stabilisator der biologischen und physikochemischen Eigenschaften (g) | 0,0001 - 0,1 |
| Stabilisator gegen mikrobielle Kontamination (g) | 0,0001 - 0,1 |
| Antioxidationsmittel (g) | 0,0001 - 0,1 |
| Natriumchlorid (g) und / oder | 0,0001 - 0,1 |
| Natriumacetat (g) | 0,0001 - 0,1 |
| Pufferlösung (ml) den Rest; | |
worin
(i) die Antihistaminika aus Anthazolin, Azelastin, Tetryzolin sind ausgewählt;
(ii) die Stabilisatoren der biologischen und physikochemischen Eigenschaften aus Macrogol 400-12000 ausgewählt sind;
(iii) der Stabilisator der Beständigkeit gegen mikrobielle Kontamination Sorbinsäure, Borsäure ausgewählt sind.

2. Ophthalmische Zusammensetzung, umfassend (in Konzentrationen pro ml der Lösung):
| | |
|---|---|
| beta-rekombinantes Interferon (IE) | 500 - 10 000 000 |
| Antihistaminika (g) | 0,0001 - 0,1 |
| Stabilisator der biologischen und physikochemischen Eigenschaften (g) | 0,0001 - 0,1 |
| Stabilisator gegen mikrobielle Kontamination (g) | 0,0001 - 0,1 |
| Antioxidationsmittel (g) | 0,0001 - 0,1 |
| Natriumacetat (g) | 0,0001 - 0,1 |
Boratpufferlösung (ml) der Rest;
worin
(i) das Antihistaminikum Azelastin ist;
(ii) der Stabilisator der biologischen und physikochemischen Eigenschaften Macrogol 400 ist;
(iii) der Stabilisator der Beständigkeit gegen mikrobielle Kontamination ist Sorbinsäure.

3. Ophthalmische Zusammensetzung, umfassend (in Konzentrationen pro ml der Lösung):
| | |
|---|---|
| gamma-rekombinantes Interferon (IE) | 500 - 10 000 000 |
| Antihistaminika (g) | 0,0001 - 0,1 |
| Stabilisator der biologischen und physikochemischen Eigenschaften (g) | 0,0001 - 0,1 |
| Stabilisator gegen mikrobielle Kontamination (g) | 0,0001 - 0,1 |
| Antioxidationsmittel (g) | 0,0001 - 0,1 |
| Natriumchlorid (g) und | 0,00005-0,05 |
| Natriumacetat (g) | 0,00005-0,05 |
Boratpufferlösung (ml) der Rest;
worin
(i) das Antihistaminikum Tetryzolin ist;
(ii) der Stabilisator der biologischen und physikochemischen Eigenschaften Macrogol 12000 ist;
(iii) der Stabilisator der Beständigkeit gegen mikrobielle Kontamination Borsäure ist.

4. Zusammensetzung nach einem der Ansprüche 1 bis 3, wobei das Antioxidationsmittel Trilon B oder Butylhydroxytoluol ist, beide in einer Konzentration von 0,0001 - 0,1 g / ml Lösung.

5. Zusammensetzung nach einem der Ansprüche 1 bis 4, wobei das Interferon rekombinantes Interferon ist, ausgewählt aus alpha-, beta-, gamma-Interferon.

6. Zusammensetzung nach einem der Ansprüche 1 bis 5, wobei die Pufferlösung ausgewählt ist aus Phosphatpuffer, Boratpuffer oder Boratacetatpuffer.

## Revendications

1. Composition ophtalmique, contenant (en concentrations par ml de solution) du:
| | |
|---|---|
| Interféron recombiné (IE) | 500 - 10 000 000 |
| Antihistaminiques (g) | 0,0001 - 0,1 |
| Stabilisateur des propriétés biologiques et physico-chimiques (g) | 0,0001 - 0,1 |
| Stabilisateur contre la contamination microbienne (g) | 0,0001 - 0,1 |
| Antioxydant (g) | 0,0001 - 0,1 |
| Chlorure de sodium (g) et / ou | 0,0001 - 0,1 |
| Acétate de sodium (g) | 0,0001 - 0,1 |
Solution tampon (ml) le reste;
dans laquelle
(i) les antihistaminiques sont choisis de l'anthazoline, l'azélastine, le tétryzoline;
(ii) les stabilisateurs des propriétés biologiques et physico-chimiques sont choisis de Macrogol 400-12000;
(iii) le stabilisateur contre la contamination microbienne est choisi d'acide sorbique, d'acide borique.

2. Composition ophtalmique, contenant (en concentrations par ml de solution) du:
| | |
|---|---|
| Interféron béta-recombiné (IE) | 500 - 10 000 000 |
| Antihistaminiques (g) | 0,0001 - 0,1 |
| Stabilisateur des propriétés biologiques et physico-chimiques (g) | 0,0001 - 0,1 |
| Stabilisateur contre la contamination microbienne (g) | 0,0001 - 0,1 |
| Antioxydant (g) | 0,0001 - 0,1 |
| Acétate de sodium(g) | 0,0001 -0,1 |
Solution de tampon borate (ml) le reste;
dans laquelle
(i) l'antihistaminique est l'azélastine;
(ii) le stabilisateur des propriétés biologiques et physico-chimiques est le Macrogol 400;
(iii) le stabilisateur de la résistance contre la contamination microbienne est l'acide sorbique.

3. Composition ophtalmique, contenant (en concentrations par ml de solution) du:
| | |
|---|---|
| Interféron gamma-recombiné (IE) | 500 - 10 000 000 |
| Antihistaminiques (g) | 0,0001 - 0,1 |
| Stabilisateur des propriétés biologiques et physico-chimiques (g) | 0,0001 - 0,1 |
| Stabilisateur contre la contamination microbienne (g) | 0,0001 - 0,1 |
| Antioxydant (g) | 0,0001 - 0,1 |
| Chlorure de sodium (g) et | 0,00005-0,05 |
| Acétate de sodium (g) | 0,00005-0,05 |
Solution tampon de borate (ml) le reste;
dans laquelle
(i) l'antihistaminique est le tétryzoline;
(ii) le stabilisateur des propriétés biologiques et physico-chimiques est le Macrogol 12000;
(iii) Le stabilisateur de la résistance contre la contamination microbienne est l'acide borique.

4. Composition correspondant à l'une des revendications 1 jusqu'à 3, où l'antioxydant est trilogue B ou butylhydroxytoluène, tout deux dans une concentration de 0,0001 - 0,1 g / ml solution.

5. Composition correspondant à l'une des revendications 1 jusqu'à 4, où l'interféron recombiné est l'interféron, choisi de l'interféron alpha, beta et gamma.

6. Composition correspondant à l'une des revendications 1 jusqu'à 5, où la solution tampon est choisi du tampon de phosphate, du tampon borate ou du tampon borate cétatique.
